(19) Europäisches Patentamt
European Patent Office
Office européen des brevets

(11) Veröffentlichungsnummer : **0 351 650 B1**

# (12) EUROPÄISCHE PATENTSCHRIFT

(45) Veröffentlichungstag der Patentschrift : **13.01.93 Patentblatt 93/02**

(51) Int. Cl.$^5$ : **C07C 61/15,** C07C 69/635, C07C 69/74

(21) Anmeldenummer : **89112308.5**

(22) Anmeldetag : **06.07.89**

## (54) Fluorcyclopropyl-Derivate.

Die Akte enthält technische Angaben, die nach dem Eingang der Anmeldung eingereicht wurden und die nicht in dieser Patentschrift enthalten sind.

(30) Priorität : **19.07.88 DE 3824433**

(43) Veröffentlichungstag der Anmeldung : **24.01.90 Patentblatt 90/04**

(45) Bekanntmachung des Hinweises auf die Patenterteilung : **13.01.93 Patentblatt 93/02**

(84) Benannte Vertragsstaaten : **BE CH DE FR GB IT LI NL**

(56) Entgegenhaltungen :
**DE-A- 2 653 189**
**US-A- 3 856 976**
**PATENT ABSTRACTS OF JAPAN, Band 9, Nr. 91 (C-277)(1814), 19. April 1985; & JP-A-59 222 430**

(73) Patentinhaber : **BAYER AG**
**W-5090 Leverkusen 1 Bayerwerk (DE)**

(72) Erfinder : **Gassen, Karl-Rudolf, Dr.**
**Auenweg 6**
**W-5068 Odenthal (DE)**
Erfinder : **Bassner, Bernd, Dr.**
**Hambergerstrasse 27 d**
**W-5090 Leverkusen 3 (DE)**

**EP 0 351 650 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Fluorcyclopropyl-Derivate, mehrere Verfahren zu deren Herstellung und deren Verwendung als Zwischenprodukte zur Synthese von Verbindungen mit fungizider Wirksamkeit.

Es sind bereits bestimmte Cyclopropyl-Derivate und deren Verwendung als Zwischenprodukte zur Herstellung von Azolyl-Derivaten mit fungiziden Eigenschaften bekannt (vgl. EP- A 0 040 345 und EP- A 0 180 136). So lassen sich 1-(4-Chlorphenoxy)-2-cyclopropyl-3-(1,2,4-triazol-1 yl)-propan-2-ol und 1-(4-Chlorphenyl)-1-(1-chlor-cycloprop-1-yl)-2-(1,2,4-triazol-1-yl)-ethan-1-ol aus entsprechenden Cyclopropyl-Derivaten herstellen und zur Bekämpfung von Pilzen verwenden. Die Wirksamkeit dieser Stoffe ist gut, läßt aber bei niedrigen Aufwandmengen in manchen Fällen zu wünschen übrig.

Aus der DE-A 2 653 189 sind halogenierte Cyclopropyl-Derivate mit insektiziden Eigenschaften bekannt, die an einem der Kohlenstoffatome des Cyclopropan-Ringes geminal durch eine Carboxyl-Gruppe und einen Phenyl-Rest substituiert sind. Entsprechende Verbindungen, in denen statt des Phenyl-Restes andere Substituenten vorhanden sind, werden jedoch nicht erwähnt.

Schließlich wurden in der US-A 3 856 976 fungizid wirksame Cyclopropancarbonsäure-Derivate beschrieben, die an einem der Kohlenstoffatome des Ringes zwei Halogenatome tragen können. Es werden aber keine Verbindungen offenbart, in denen ein Fluoratom am Cyclopropyl-Ring enthalten ist.

Es wurden nun neue Fluorcyclopropyl-Derivate der Formel

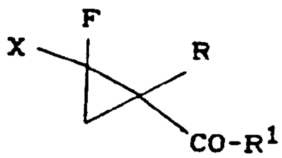

(I)

in welcher

R für Alkyl mit 1 bis 4 Kohlenstoffatomen oder für Benzyl steht, das im Phenylteil einfach bis dreifach, gleichartig oder verschieden substituiert sein kann durch Halogen, Alkyl mit 1 bis 4 Kohleastoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halogen substituiertes Phenyl oder gegebenenfalls durch Alkyl mit 1 oder 2 Kohlenstoffatomen und/oder Halogen substituiertes Phenoxy,

X für Wasserstoff, Chlor oder Brom steht und

$R^1$ für Hydroxy, Alkoxy mit 1 bis 6 Kohlenstoffatomen, Alkyl mit 1 bis 6 Kohlenstoffatomen, Fluor, Chlor Brom oder Iod steht,

gefunden.

Weiterhin wurde gefunden, daß sich Fluorcyclopropyl-Derivate der Formel (I) herstellen lassen, indem man

a) Vinyl-cyclopropan-Derivate der Formel

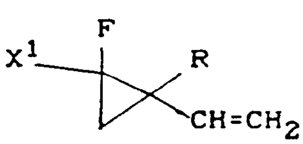

(II)

in welcher

R die oben angegebene Bedeutung hat und

$X^1$ für Chlor oder Brom steht,

mit starken oxidationsmitteln in Gegenwart eines Verdünnungsmittels umsetzt,

oder

b) Fluorcyclopropyl-Derivate der Formel

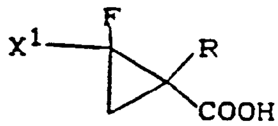

(Ia)

in welcher
R und $X^1$ die oben angegebene Bedeutung haben,
entweder

α) mit einem Halogenierungsmittel gegebenenfalls in Gegenwart eines Verdünnungsmittels umsetzt,
oder

β) mit Alkoholen der Formel

$$R^2\text{-OH} \quad \text{(III)}$$

in welcher
$R^2$ für Alkyl mit 1 bis 6 kohlenstoffatomen steht,
gegebenenfalls in Gegenwart eines Katalysators und gegebenenfalls in Gegenwart eines Verdünnungsmittels umsetzt,
oder

γ) mit Wasserstoff in Gegenwart eines Katalysators und in Gegenwart eines Amins sowie in Gegenwart eines Verdünnungsmittels umsetzt,
oder

c) Fluorcyclopropyl-Derivate der Formel

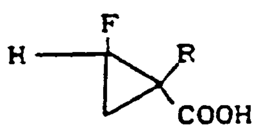

(Ib)

in welcher
R die oben angegebene Bedeutung hat,
mit einem Halogenierungsmittel gegebenenfalls in Gegenwart eines Verdünnungsmittels umsetzt,
oder

d) Fluorcyclopropyl-Derivate der Formel

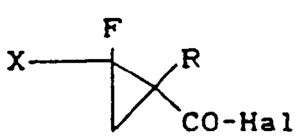

(Ic)

in welcher
R und X die oben angegebene Bedeutung haben und
Hal für Fluor, Chlor oder Brom steht,
mit Alkoholen der Formel

$$R^2\text{-OH} \quad \text{(III)}$$

in welcher
$R^2$ die oben angegebene Bedeutung hat,
gegebenenfalls in Gegenwart eines Katalysators und gegebenenfalls in Gegenwart eines Verdünnungsmittels umsetzt,
oder

e) Fluorcyclopropyl-Derivate der Formel

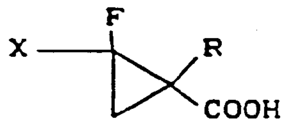

(Id)

in welcher
R und X die oben angegebene Bedeutung haben,
mit metallorganischen Verbindungen der Formel

$R^3$-Li (IV)

in welcher
$R^3$ für Alkyl mit 1 bis 6 kohlenstoffatomen steht,
in Gegenwart eines Verdünnungsmittels umsetzt.

Schließlich wurde gefunden, daß sich die neuen Fluorcyclopropyl-Derivate der Formel (I) sehr gut als Zwischenprodukte zur Herstellung von Fluorcyclopropylhydroxyethyl-azolen mit fungizider Wirksamkeit verwenden lassen.

Überraschenderweise zeigen die aus den erfindungsgemäßen Fluorcyclopropyl-Derivaten der Formel (I) herstellbaren Fluorcyclopropyl-hydroxyethyl-azole eine bessere fungizide Wirksamkeit als 1-(4-Chlorphenoxy)-2-cyclopropyl -3-(1,2,4-triazol-1-yl)-propan-2-ol und 1-(4-Chlorphenyl )-1-(1-chlor-cycloprop-1-yl)-2-(1,2,4-triazol -1-yl)-ethan-1-ol, welches konstitutionell ähnliche, vorbekannte Wirkstotte gleicher Wirkungsrichtung sind.

Bevorzugt sind diejenigen Fluorcyclopropyl-Derivate der Formel (I), in denen
R für Methyl, Ethyl, Isopropyl, tert.-Butyl steht,
oder für Benzyl steht, das im Phenylteil einfatch oder zweifach, gleichartig oder verschieden substituiert sein kann durch Fluor, Chlor und/oder Methyl,
X für Wasserstoff, Chlor oder Brom steht und
$R^1$ für Hydroxy, Alkoxy mit 1 bis 4 Kohlenstoffatomen, Alkyl mit 1 bis 4 Kohlenstoffatomen, fluor, Chlor, Brom oder Iod steht.

Besonders bevorzugt sind diejenigen Fluorcyclopropyl-Derivate der Formel (I), in denen
R für Methyl, Ethyl, gegebenenfalls einfach oder zweifach durch Fluor, Chlor und/oder Methyl substituiertes Benzyl steht,
X für Wasserstoff, Chlor oder Brom steht und
$R^1$ für Hydroxy, Methoxy, Ethoxy, Isopropoxy, n-Butoxy, Methyl, Ethyl, Chlor, Brom oder Iod steht.

Verwendet man 2-Fluor-2-chlor-1-methyl-1-vinyl-cyclopropan als Ausgangsstoff und Kaliumpermanganat als Oxidationsmittel, so kann der Verlauf des erfindungsgemäßen Verfahrens (a) durch das folgende Formelschema veranschaulicht werden:

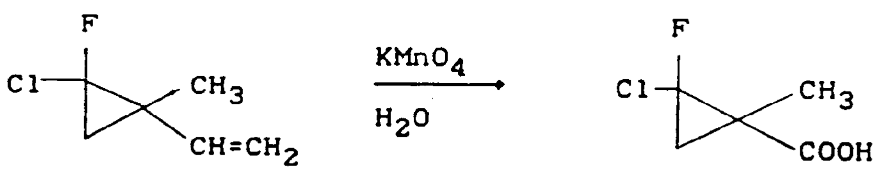


Verwendet man 2-Fluor-2-chlor-1-methyl-cyclopropan-carbonsäure als Ausgangsstoff und Thionylchlorid als Halogenierungsmittel, so kann der Verlauf des erfindungsgemäßen Verfahrens (b, Variante α) durch das folgende Formelschema veranschaulicht werden:

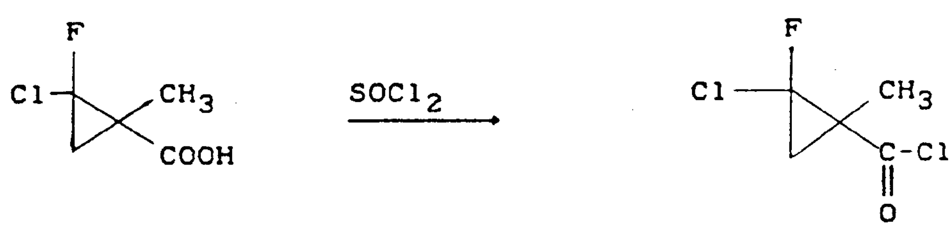

Verwendet man 2-Fluor-2-chlor-1-methyl-cyclopropancarbonsäure und Ethanol als Ausgangsstoffe und Schwefelsaüre als Katalysator, so kann der Verlauf des erfindungsgemäßen Verfahrens (b, Variante β) durch das folgende Formelschema veronschaulicht werden:

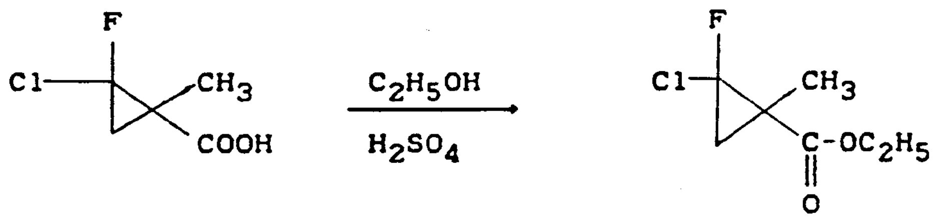


Verwendet man 2-Fluor-2-chlor-1-methyl-cyclopropan-carbonsäure als Ausgangsstoff und Wasserstoff in Gegenwart von Raney-Nickel sowie 1,2-Diamino-ethan als Reaktionskomponenten, so kann der Verlauf des erfindungsgemäßen Verfahrens (b, Varinate γ) durch das folgende Formelschema veranschaulicht werden:

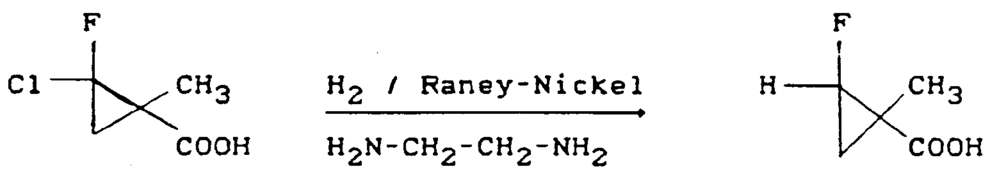


Verwendet man 2-Fluor-1-methyl-cyclopropan-carbonsäure als Ausgangsstoff und Thionylchlorid als Halogenierungsmittel, so kann der Verlauf des erfindungsgemäßen Verfahrens (c) durch das folgende Formelschema veranschaulicht werden:

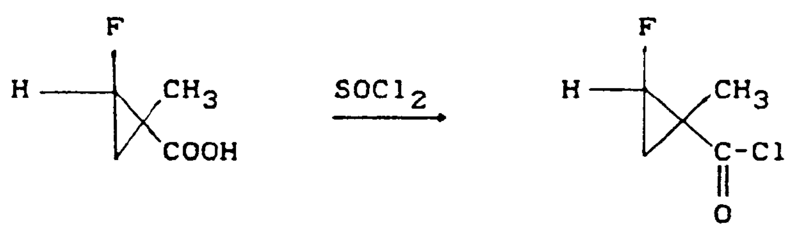


Verwendet man 2-Fluor-1-methyl-cyclopropan-carbonsäurechlorid als Ausgangsstoff und Ethanol als Reaktionskomponente, so kann der Verlauf des erfindungsgemäßen Verfahrens (d) durch das folgende Formelschema veranschaulicht werden:

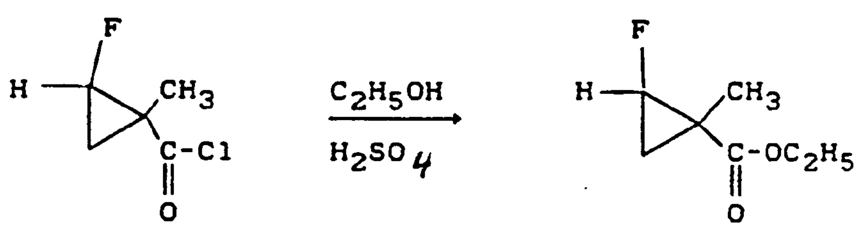


Verwendet man 2-Fluor-2-chlor-1-methyl-cyclopropancarbonsäure als Ausgangsstoff und Methyl-lithium als metallorganische Verbindung, so kann der Verlauf des erfindungsgemäßen Verfahrens (e) durch das folgende Formelschema veranschaulicht werden:

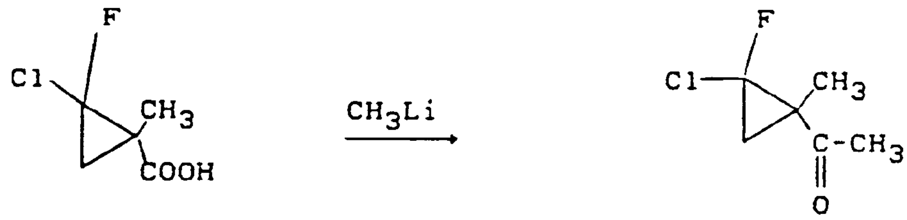


Die bei der Durchführung des erfindungsgemäßen Verfahrens (a) als Ausgangsstoffe benötigten Vinylcyclopropan-Derivate sind durch die Formel (II) allgemein definiert. In dieser Formel steht R vorzugsweise für diejenigen Reste, die bereits im Zusammenhang mit der Beschreibung der erfindungsgemäßen Stoffe der Formel (I) vorzugsweise für R genannt wurden. $X^1$ steht für Chlor oder Brom.

Die Vinylcyclopropan-Derivate der Formel (II) sind bekannt oder lassen sich nach prinzipiell bekannten Verfahren herstellen (vgl. Liebigs Ann. Chem. 710, 17-35 (1967)).

Als starke Oxidationsmittal kommen bei der Durchführung des erfindungsgemäßen Verfahrens (a) alle diejenigen Oxidationsmittel in Betracht, die zur Spaltung von olefinischen Doppelbindungen geeignet sind. Vorzugsweise verwendbar ist Kaliumpermanganat.

Als Verdünnungsmittel kommen bei der Durchführung des erfindungsgemäßen Verfahrens (a) alle für derartige Umsetzungen üblichen Lösungsmittel in Frage. Vorzugsweise verwendbar ist Wasser.

Die Temperaturen können bei der Durchführung des erfindungsgemäßen Verfahrens (a) innerhalb eines bestimmten Bereiches variiert werden. Im allgemeinen arbeitet man bei Temperaturen zwischen 0°C und 60° C, vorzugsweise zwischen 10° C und 50° C.

Bei der Durchführung des erfindungsgemäßen Verfahrens (a) arbeitet man ebenso wie bei den anderen in dieser Anmeldung beschriebenen Verfahren im allgemeinen unter Normaldruck. Es ist jedoch jeweils auch möglich, unter erhöhtem oder vermindertem Druck zu arbeiten.

Bei der Durchführung des erfindungsgemäßen Verfahrens (a) setzt man auf 1 Mol an Vinylcyclopropan-Derivat der Formel (II) im allgemeinen 2 bis 3 Mol an starkem Oxidationsmittel ein. Die Aufarbeitung erfolgt nach üblichen Methoden.

Die bei der Durchführung des erfindungsgemäßen Verfahrens (b) als Ausgangsstoffe benötigten Fluorcyclopropyl-Derivate sind durch die Formel (Ia) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden. $X^1$ steht für Chlor oder Brom.

Die Fluorcyclopropyl-Derivate der Formel (Ia) lassen sich nach dem erfindungsgemäßen Verfahren (a) herstellen.

Als Halogenierungsmittel kommen bei der Durchführung des erfindungsgemäßen Verfahrens (b, Variante $\alpha$) alle diejenigen Stoffe in Frage, die zur Umwandlung von Säuren in Säurehalogenide geeignet sind. Vorzugsweise verwendbar sind Thionylchlorid, Sulfurylchlorid, Phosphortrichlorid, Thionylbromid und Sulfurylbromid. Die Säurefluoride und Säureiodide lassen sich aus den entsprechenden Bromiden oder Chloriden nach üblichen Methoden herstellen.

Als Verdünnungsmittel kommen bei der Durchführung des erfindungsgemäßen Verfahrens (b, Variante $\alpha$) alle für derartige Reaktionen üblichen inerten organischen Solventien in Betracht. Vorzugsweise wird das jeweilige Halogenierungsmittel gleichzeitig auch als Verdünnungsmittel benutzt.

Die Reaktionstemperaturen können bei der Durchführung des erfindungsgemäßen Verfahrens (b, Variante $\alpha$) innerhalb eines größeren Bereiches variiert werden. Im allgemeinen arbeitet man bei Temperaturen zwischen 0° C und 150° C, vorzugsweise zwischen 20° C und 80° C.

Bei der Durchführung des erfindungsgemäßen Verfahrens (b, Variante $\alpha$) setzt man auf 1 Mol Fluorcyclopropyl-Derivat der Formel (Ia) 1 bis 2 Äquivalente oder auch einen größeren Überschuß an Halogenierungsmittel ein. Die Aufarbeitung erfolgt nach üblichen Methoden. Zur Herstellung von Säureioden setzt man Säurebromide mit Kaliumiodid um; während Säurefluoride aus anderen Säurehalogeniden durch Umsetzung mit Fluoriden, wie z.B. Natriumfluorid, Kaliumfluorid, Cäsiumfluorid oder Ammoniumfluorid, oder durch Umsetzung mit Fluorwasserstoffsäure zugänglich sind.

Die bei der Durchführung des erfindungsgemäßen Verfahrens (b, Variante $\beta$) als Reaktionskomponenten benötigten Alkohole sind allgemein bekannte Verbindungen der organischen Chemie.

Als Katalysatoren kommen bei der Durchführung des erfindungsgemäßen Verfahrens (b, Variante ß) alle für die Herstellung von Estern aus Säuren oder Säurehalogeniden üblichen Reaktionsbeschleuniger in Frage. Vorzugsweise verwendbar sind anorganische Säuren, wie Schwefelsäure, oder auch starke organische Säuren, wie p-Toluolsulfonsäure. Ebenfalls verwendbar sind anorganische Basen, wie Natriumhydrogencarbonat

oder Natriumhydroxid, oder auch organische Basen, wie Pyridin oder tertiäre Amine.

Als Verdünnungsmittel kommen bei der Durchführung des erfindungsgemäßen Verfahrens (b, Variante β) alle für derartige Umsetzungen üblichen organischen Solventien in Betracht. Vorzugsweise fungiert im Überschuß eingesetzter Alkohol der Formel (III) gleichzeitig als Verdünnungsmittel.

Die Reaktionstemperaturen können bei der Durchführung des erfindungsgemäßen Verfahrens (b, Variante β) innerhalb eines größeren Bereiches variiert werden. Im allgemeinen arbeitet man bei Temperaturen zwischen 0° C und 150° C, vorzugsweise zwischen 20° C und 140° C.

Bei der Durchführung des erfindungsgemäßen Verfahrens (b, Variante β) setzt man auf 1 Mol an Fluorcyclopropyl-Derivat der Formel (Ia) vorzugsweise 1 bis 3 Mol oder auch einen größeren Überschuß an Alkohol der Formel (III) sowie eine katalytische Menge an Reaktionsbeschleuniger ein. die Aufarbeitung erfolgt nach üblichen Methoden.

Als Katalysatoren kommen bei der Durchführung des erfindungsgemäßen Verfahrens (b, Variante γ) alle für derartige Hydrierungen üblichen Reaktionsbeschleuniger in Frage. Vorzugsweise verwendbar sind Metallkatalysatoren, wie Raney-Nickel.

Als Amine kommen bei der Durchführung des erfindungsgemäßen Verfahrens (b, Variante γ) alle für derartige Umsetzungen üblichen Amine in Betracht. Vorzugsweise verwendbar ist 1,2-Diamino-ethan.

Als Verdünnungsmittel kommen bei der Durchführung des erfindungsgemäßen Verfahrens (b, Variante γ) alle für derartige Umsetzungen üblichen organischen Solventien in Frage. Vorzugsweise verwendbar sind Alkohole, wie Methanol oder Ethanol, und außerdem Ether, wie Diethylether, Dioxan oder Tetrahydrofuran.

Bei der Durchführung des erfindungsgemäßen Verfahrens (b, Variante γ) arbeitet man im allgemeinen unter einem Wasserstoff-Druck zwischen 5 und 30 bar, vorzugsweise zwischen 10 und 25 bar.

Die Reaktionstemperaturen können bei der Durchführung des erfindungsgemäßen Verfahrens (b, Variante γ) innerhalb eines größeren Bereiches variiert werden. Im allgemeinen arbeitet man bei Temperaturen zwischen 20° C und 120° C, vorzugsweise zwischen 40° C und 100° C.

Bei der Durchführung des erfindungsgemäßen Verfahrens (b, Variante γ) setzt man auf 1 Mol an Fluorcyclopropyl-Derivat der Formel (Ia) im allgemeinen 2 bis 4 Mol an Amin sowie die jeweils benötigte Menge an Katalysator ein und hydriert mehrere Stunden unter erhöhtem Druck mit Wasserstoff. Die Aufarbeitung erfolgt nach üblichen Methoden.

Die bei der Durchführung des erfindungsgemäßen Verfahrens (c) als Ausgangsstoffe benötigten Fluorcyclopropyl-Derivate sind durch die Formel (Ib) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden.

Die Fluorcyclopropyl-Derivate der Formel (Ib) lassen sich nach dem erfindungsgemäßen Verfahren (b, Variante γ) herstellen.

Als Halogenierungsmittel kommen bei der Durchführung des erfindungsgemäßen Verfahrens (c) alle diejenigen Stoffe in Betracht, die bereits im Zusammenhang mit der Beschreibung des erfindungsgemäßen Verfahrens (b, Variante α) als Halogenierungsmittel genannt wurden.

Im übrigen entsprechen die Reaktionsbedingungen bei der Durchführung des erfindungsgemäßen Verfahrens (c) völlig denen, die bei dem erfindungsgemäßen Verfahren (b, Variante α) erwähnt wurden.

Die bei der Durchführung des erfindungsgemäßen Verfahrens (d) als Ausgangsstoffe benötigten Fluorcyclopropyl-Derivate sind durch die Formel (Ic) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für den Rest R genannt wurden. Hal steht für Fluor, Chlor oder Brom, und X steht für Wasserstoff, Chlor oder Brom.

Die Fluorcyclopropyl-Derivate der Formel (Ic) lassen sich nach den erfindungsgemäßen Verfahren (b, Variante α) und (c) herstellen.

Als Alkohole bzw. Katalysatoren kommen bei der Durchführung des erfindungsgemäßen Verfahrens (d) alle diejenigen Stoffe in Betracht, die bereits im Zusammenhang mit der Beschreibung des erfindungsgemäßen Verfahrens (b, Variante β) als Alkohole bzw. Reaktionsbeschleuniger genannt wurden.

Im übrigen entsprechen die Reaktionsbedingungen bei der Durchführung des erfindungsgemäßen Verfahrens (d) völlig denen, die bei dem erfindungsgemäßen Verfahren (b, Variante β) erwähnt wurden.

Die bei der Durchführung des erfindungsgemäßen Verfahrens (e) als Ausgangsstoffe benötigten Fluorcyclopropyl-Derivate sind durch die Formel (Id) allgemein definiert. In dieser Formel haben R und X vorzugsweise diejenigen Bedeutungen, die bereits im Zusammenhang mit der Beschreibung der erfindungsgemäßen Stoffe der Formel (I) vorzugsweise für die Reste R und X genannt wurden.

Die Fluorcyclopropyl-Derivate der Formel (Id) lassen sich nach den erfindunggemäßen Verfahren (a) und (b, Variante γ) herstellen.

Die bei der Durchführung des erfindungsgemäßen Verfahrens (e) als Reaktionskomponenten benötigten

metallorganischen Verbindungen sind allgemein bekannte Verbindungen der organischen Chemie.

Als Verdünnungsmittel kommen bei der Durchführung des erfindungsgemäßen Verfahrens (e) alle für derartige Umsetzungen üblichen inerten organischen Solventien in Frage. Vorzugsweise verwendbar sind Ether, wie Diethylether, Dioxan oder Tetrahydrofuran.

Die Reaktionstemperaturen können bei der Durchführung des erfindungsgemäßen Verfahrens (e) innerhalb eines größeren Bereiches variiert werden. Im allgemeinen arbeitet man bei Temperaturen zwischen -100° C und +50° C, vorzugsweise zwischen -78° C und +0° C.

Bei der Durchführung des erfindungsgemäßen Verfahrens (e) arbeitet man unter Schutzgasatmosphäre, wie z.B. unter Argon oder Stickstoff.

Bei der Durchführung des erfindungsgemäßen Verfahrens (e) setzt man auf 1 Mol an Fluorcyclopropyl-Derivat der Formel (Id) im allgemeinen 1,5 bis 3,0 Mol, vorzugsweise 2,0 Mol an metallorganischer Verbindung der Formel (IV) ein. Die Aufarbeitung erfolgt nach üblichen Methoden. Im allgemeinen geht man so vor, daß man das Reaktionsgemisch auf Eis und anorganische Säure gibt, die organische Phase abtrennt, die wäßrige Phase mit einem mit Wasser wenig mischbaren organischen Lösungsmittel extrahiert, die vereinigten organischen Phasen trocknet und destilliert.

Die erfindungsgemäßen Fluorcyclopropyl-Derivate der Formel (I) eignen sich als Zwischenprodukte zur Synthese von Pflanzenschutzmitteln, insbesondere zur Herstellung von Stoffen mit fungizider Wirksamkeit (vgl. EP-A 0351 646 und EP-A 0 351 649).

So lassen sich z.B. Fluorcyclopropyl-hydroxy-ethyl-triazole der Formel

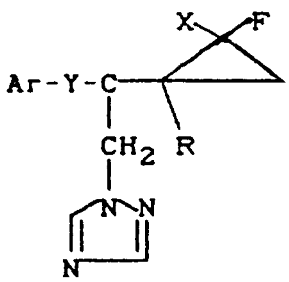

(V)

in welcher

R und

X die oben angegebene Bedeutung haben,

Ar für gegebenenfalls substituiertes Aryl steht und

γ für die Gruppierungen $-OCH_2-$, $-SCH_2-$, $-CH_2-CH_2-$ oder $-CH=CH-$ steht,

herstellen, indem man

f) Methyl-cyclopropyl-ketone der Formel

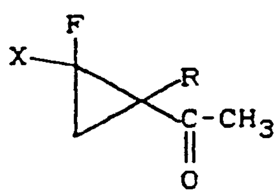

(Ie)

in welcher

R und X die oben angegebene Bedeutung haben,

mit Chlorierungsmitteln oder Bromierungsmitteln, wie Sulfurylchlorid, Sulfurylbromid oder Brom, in Gegenwart eines Verdünnungsmittels, wie Methylenchlorid, Chloroform oder Tetrachlorkohlenstoff, bei Temperaturen zwischen -10° C und +60° C, vorzugsweise zwischen 0° C und 40° C, umsetzt und die dabei entstehenden Halogenketone der Formel

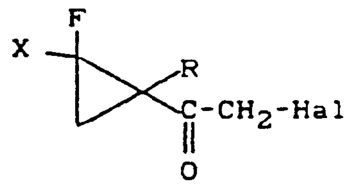

(VI)

in welcher
R und X die oben angegebene Bedeutung haben,
und
Hal für Chlor oder Brom steht,
mit Verbindungen der Formel

Ar-Z-H (VII)

in welcher
Ar die oben angegebene Bedeutung hat und
Z für Sauerstoff oder Schwefel steht,

in Gegenwart eines Säurebindemittels und gegebenenfalls in Gegenwart eines Verdünnungsmittels bei Temperaturen zwischen 0° C und 150° C, vorzugsweise zwischen 20° C und 130° C, umsetzt und die dabei entstehenden Cyclopropyl-ketone der Formel

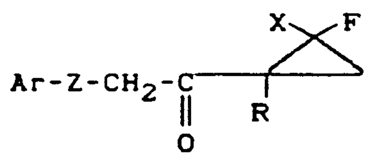

(VIII)

in welcher
Ar, R, X und Z die oben angegebene Bedeutung haben,
entweder

α) mit Dimethyloxosulfonium-methylid der Formel

$$\overset{\delta^{\oplus}\delta^{\ominus}}{(CH_3)_2SOCH_2} \qquad (IX)$$

oder

β) mit Dimethylsulfonium-methylid der Formel

$$\overset{\delta^{\oplus}\delta^{\ominus}}{(CH_3)_2S\ CH_2} \qquad (X)$$

in Gegenwart eines Verdünnungsmittels bei Temperaturen zwischen 0° C und 100° C, vorzugsweise zwischen 10° C und 60° C, umsetzt und schließlich die dabei entstehenden Oxirane der Formel

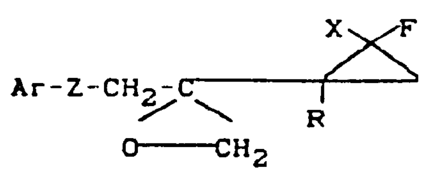

(XI)

in welcher
Ar, R, X und Z die oben angegebene Bedeutung haben,
mit 1,2,4-Triazol der Formel

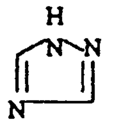

(XII)

in Gegenwart eines Säurebindemittels und in Gegenwart eines Verdünnungsmittels bei Temperaturen zwischen 0° C und 200° C, vorzugsweise zwischen 50° C und 150° C, umsetzt,
oder indem man

g) Methyl-cyclopropyl-ketone der Formel

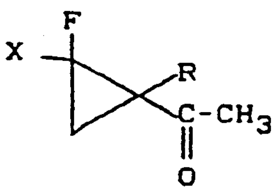

(Ie)

in welcher
R und X die oben angegebene Bedeutung haben,
mit Aldehyden der Formel

Ar-CHO (XIII)

in welcher
Ar die oben angegebene Bedeutung hat,
in Gegenwart eines Katalystors, wie Natriumhydroxid oder Kaliumhydroxid, sowie in Gegenwart eines Verdünnungsmittels, wie Methanol, Ethanol, Isopropanol, n-Butanol oder tert.-Butanol, bei Temperaturen zwischen 0° C und 100° C, vorzugsweise zwischen 10° C und 80° C, umsetzt und gegebenenfalls die dabei entstehenden Cyclopropyl-ketone der Formel

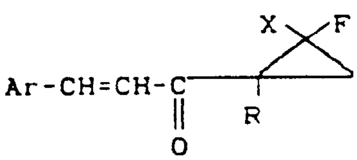

(XIV)

in welcher
R, X und Ar die oben angegebene Bedeutung haben,
in Gegenwart eines Hydrierkatalysators und in Gegenwart eines Verdünnungsmittels mit Wasserstoff hydriert und schließlich die so erhaltenen Cyclopropyl-ketone der Formel

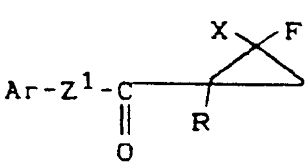

(XV)

in welcher
Ar, X und R die oben angegebenen Bedeutungen haben und
$Z^1$ für die Gruppierung $-CH_2-CH_2-$ steht,
α) mit Dimethyloxosulfonium-methylid der Formel

$$\overset{\delta^{\oplus}\delta^{\ominus}}{(CH_3)_2SOCH_2} \qquad (IX)$$

oder

β) mit Dimethylsulfonium-methylid der Formel

$$\overset{\delta^{\oplus}\delta^{\ominus}}{(CH_3)_2S\ CH_2} \qquad (X)$$

in Gegenwart eines Verdünnungsmittels bei Temperaturen zwischen 0° C und 100° C, vorzugsweise zwischen 10° C und 60° C, umsetzt und schließlich die dabei entstehenden Oxirane der Formel

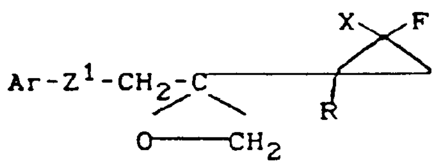

(XVI)

in welcher

Ar, R, X und $Z^1$ die oben angegebene Bedeutung haben,

mit 1,2,4-Triazol der Formel

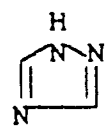

(XII)

in Gegenwart eines Säurebindemittels und in Gegenwart eines Verdünnungsmittels bei Temperaturen zwischen 0° C und 20° C, vorzugsweise zwischen 50° C und 150° C, umsetzt.

Weiterhin lassen sich Hydroxyalkinyl-azolyl-Derivate der Formel

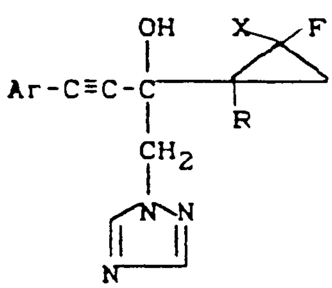

(XVII)

in welcher

Ar, X und R die oben angegebene Bedeutung haben,

herstellen, indem man

h) Fluorcyclopropyl-Derivate der Formel

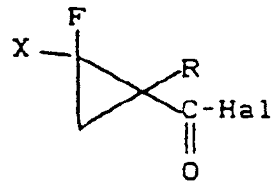

(Ic)

in welcher
R, X und Hal die oben angegebene Bedeutung haben,
mit Acetyl-Derivaten der Formel

Ar-C≡CH (XVIII)

in welcher
Ar die oben angegebene Bedeutung hat,
in Gegenwart eines Katalysators, wie Kupfer-(I)-bromid, und in Gegenwart eines Säurebindemittels, wie Natriumhydroxid oder Kaliumhydroxid, und in Gegenwart eines Verdünnungsmittels, wie Toluol, bei Temperaturen zwischen 0° C und 150° C, vorzugsweise zwischen 20° C und 120° C, umsetzt und die dabei entstehenden Cyclopropylketone der Formel

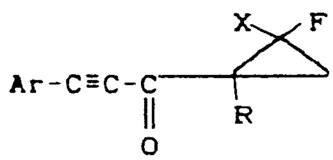

(XIX)

in welcher
Ar, X und R die oben angegebene Bedeutung haben,
α) mit Dimethylsulfonium-methylid der Formel

$$\overset{\delta^{\oplus}}{(CH_3)_2S}\ \overset{\delta^{\ominus}}{CH_2} \qquad (X)$$

in Gegenwart eines Verdünnungsmittels bei Temperaturen zwischen 0° C und 100° C, vorzugsweise zwischen 10° C und 60° C, umsetzt und schließlich die dabei entstehenden Oxirane der Formel

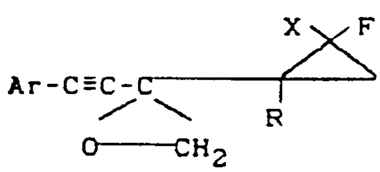

(XX)

in welcher
Ar, X und R die oben angegebene Bedeutung haben,
mit 1,2,4-Triazol der Formel

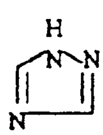

(XII)

in Gegenwart eines Säurebindemittels und in Gegenwart eines Verdünnungsmittels bei Temperaturen zwischen 0° C und 200° C, vorzugsweise zwischen 50° C und 150° C, umsetzt.

Die übrigen Fluorcyclopropyl-Derivate der Formel (I) lassen sich in entsprechender Weise als Zwischenprodukt zur Synthese von Pflanzenschutzmitteln, insbesondere von Stoffen mit fungizider Wirksamkeit verwenden.

Die Fluorcyclopropyl-hydroxyethyl-triazole der Formel (V) und die Hydroxalkinyl-azolyl-Derivate der Formel (XVII) weisen eine starke mikrobizide Wirkung auf und können als Fungizide eingesetzt werden (vgl. EP-A 0 351 646 und EP-A 0 351 649).

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe der Formeln (V) und (XVII) in den zur Bekämpfung von Pflanzenkrankheiten notwendigen Konzentrationen erlaubt eine Behandlung von oberirdischen Pflanzenteilen, von Pflanz- und Saatgut und des Bodens.

Die Wirkstoffe der Formeln (V) und (XVII) eignen sich insbesondere zur Bekämpfung von Getreidekrankheiten, wie Erysiphe graminis, Puccinia recondita, Cochliobolus sativus, Pyrenophora teres, Leptosphaeria nodorum und Gerstenmehltau; ferner von Reiskranheiten, wie Pyricularia oryzae und Pellicularia sasakii; sowie von Venturia-Arten und Gurkenmehltau. Die Stoffe besitzen außerdem eine sehr gute in-vitro-Wirkung. Die Wirkstoffe der Formeln (V) und (XVII) können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wassers mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol -Ether, z.B. Alkylarylpolyglycol-Ether, Alkylsulfonate, Alkylsulfate, Arylsulfonate sowie Eiweißhydrolysate; als Dispergiermittel kommen in Frage: z.B. Lignin-sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecitine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe der Formeln (V) und (XVII) können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe der Formeln (V) und (XVII) können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Die Aufwandmenge an Wirkstoffen der Formeln (V) und (XVII) kann je nach Art der Applikation in einem größeren Bereich variiert werden. So liegen die Wirkstoffkonzentrationen bei der Behandlung von Pflanzeateilen in den Aawendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung der erfindungsgemäßen 2,2-Difluorcyclopropyl-Derivate der Formel (I) geht aus den folgenden Beispielen hervor.

Herstellungsbeispiele

Beispiel 1

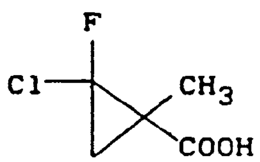

(I-1)

122,5 g (0,91 Mol) 2-Fluor-2-chlor-1-methyl-1-vinyl-cyclo/propan in 10 l Wasser werden portionsweise mit 474 g (3 Mol) Kaliumpermanganat versetzt. Man läßt 36 Stunden bei Raumtempertur rühren, filtriert von Braunstein ab und wäscht gut mit Wasser nach. Das Filtrat wird mit konzentrierter Salzsäure sauer gestellt und mit Dichlormethan extrahiert. Nach dem Trocknen der organischen Phase wird das Lösungsmittel unter vermindertem Druck entfernt und der Rückstand destilliert.

Man erhält so 115 g (82 % der Theorie) 2-Fluor-2-chlor -1-methylcyclopropancarbonsäure vom Siedepunkt

77-78° C/0,1 mbar.

Beispiel 2

Nach der im Beispiel 1 angegebenen Methode wird auch die Verbindung der Formel

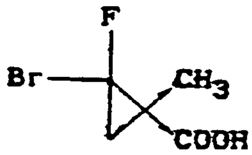

(I-2)

erhalten.
Siedepunkt: 45-50°C/0,01 mbar
Herstellung des Ausgangssustanz der Formel

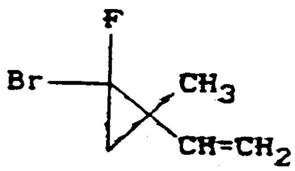

(II-1)

Zu einer intensiv gerührten Mischung aus 7,5 g (0,11 Mol) Isopren, 19 g (0,1 Mol) Fluor-dibrom-methan, 0,5 g (4 mMol) Tetrabutylammonium-bromid und 10 ml Dichlormethan werden 20 ml 50 %-ige wäßrige Natronlauge so zugetropft, daß das Reaktionsgemisch unter starkem Rückfluß siedet. Man rührt nach beendeter Zugabe noch fünf Stunden bei Raumtemperatur, gießt dann das Reaktionsgemisch auf Wasser, extrahiert mehrfach mit Diethylether und wäscht die vereinigten organischen Phasen nacheinander mit verdünnter wäßriger Salzsäure und verdünnter wäßriger Natriumhydrogencarbonat-Lösung. Nach dem Trocknen wird unter vermindertem Druck destilliert. Man erhält auf diese Weise 13,2 g (74 % der Theorie) an 2-Fluor-2-brom-1-methyl-1-vinyl-cyclopropan (cis/trans-Gemisch ca. 1:1) in 0,01 mbar.

Beispiel 3

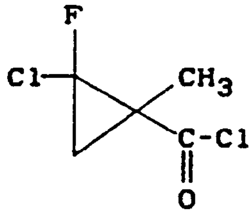

(I-3)

Ein Gemisch aus 51 g (0,33 Mol) 2-Fluor-2-chlor-1-methylcyclopropancarbonsäure und 60 g (0,5 Mol) Thionylchlorid wird in einer Destillationsapparatur unter Rühren langsam erhitzt, wobei zuerst überschüssiges Thionylchlorid und dann das gewünschte Produkt überdestilliert. Man erhält auf diese Weise 54 g (97 %, der Theorie) an 2-Fluor-2-chlor-1-methyl-cyclopropancarbonsäure-chlorid in Form einer Flüssigkeit vom Siedepunkt 82-85° C/100 mbar.

Beispiel 4

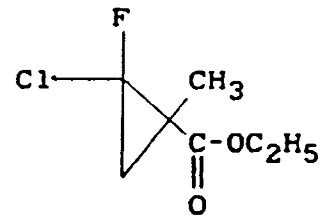

(I-4)

Unter Rühren werden bei Raumtemperatur 44 ml Ethanol zu 43 g (0,28 Mol) 2-Fluor-2-chlor-1-methyl-cyclopropancarbonsäure gegeben. Nach Zugabe von Schwefelsäure wird das Reaktionsgemisch 16 Stunden unter Rückfluß erhitzt. Anschließend wird überschüssiges

Ethanol abdestilliert, der Rückstand getrocknet und unter vermindertem Druck destilliert. Man erhält auf diese Weise 40 g (79 % der theorie) an 2-Fluor-2-chlor -1-methyl-cyclopropan-carbonsäure-ethylester in Form einer Flüssigkeit vom Siedepunkt 62-63° C/17 mbar.

Beispiel 5

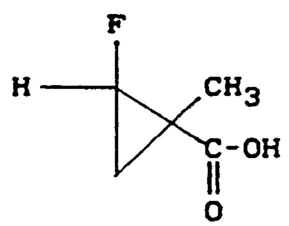

(I-5)

38 g (0,25 Mol) 2-Fluor-2-chlor-1-methyl-cyclopropancarbonsäure (cis/trans-Gemisch) und 30 g (0,5 Mol) 1,2-Diaminoethan werden in 750 ml Ethanol gelöst. Nach Zugabe von 10 g Raney-Nickel wird 8 Stunden unter einem Wasserstoffdruck von 20 bar bei 80° C hydriert. Der Katalysator wird abfiltriert und das Filtrat wird nacheinander mit verdünnter, wäßriger Salzsäure und mit Wasser gewaschen, danach getrocknet und destilliert. Man erhält auf diese Weise 21 g (71 % der theorie) an 2-Fluor-1-methyl-cyclopropan-carbonsäure (cis/trans-Gemisch) in Form einer Flüssigkeit vom Siedepunkt 52-54° C bei 0,1 mbar.

Beispiel 6

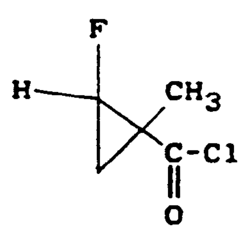

(I-6)

Ein Gemisch aus 51 g (0,43 Mol) 2-Fluor-1-methyl-cyclopropan-carbonsäure (cis/trans-Gemisch) und 100 ml Thionylchlorid wird in einer Destillationsapparatur unter Rühren langsam erhitzt, wobei zuerst überschüssiges Thionylchlorid und dann das gewünschte Produkt überdestilliert. Man erhält auf diese Weise 56 g (93 % der Theorie) an 2-Fluor-1-methyl-cyclopropan-carbonsäurechlorid (cis/trans-Gemisch) in Form einer Flüssigkeit vom Siedepunkt 40-42° C bei 20 mbar.

Beispiel 7

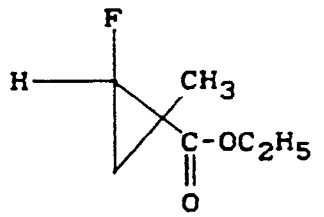

(I-7)

Unter Rühren bei 20° C werden 40 ml Ethanol zu 20 g (0,12 Mol) 2-Fluor-1-methyl-cyclopropan-carbonsäurechlorid (cis/trans-Gemisch) getropft. Nach Zugabe von 2 Tropfen konzentrierter Schwefelsäure wird das Reaktionsgemisch nach eine Stunde unter Rückfluß erhitzt.

Anschließend wird überschüssiges Ethanol abdestilliert, der Rückstand getrocknet und unter vermindertem Druck destilliert. Man erhält auf diese Weise 18,4 g (85 % der Theorie) an 2-Fluor-1-methyl-cyclopropancarbonsäure-ethylester (cis/trans-Gemisch) in Form einer Flüssigkeit vom Siedepunkt 42-45° C bei 18 mbar.

$n_D^{20}$ = 1,4069

## Patentansprüche

1. Fluorcyclopropyl-Derivate der Formel

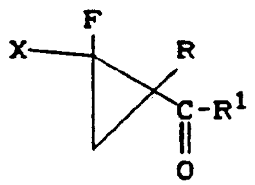

(I)

in welcher
R für Alkyl mit 1 bis 4 Kohlenstoffatomen oder
für Benzyl steht, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogonalkyl mit 1 oder 2 Kohlonstoffatomen und 1 bis 5 gleichen oder verschiedenen Halogenatomen, Halogonalkoxy mit 1 oder 2 Kohlenstoffatomen
und 1 bis 5 gleichen oder vorschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlonstoffatomen und/oder Halogen substituiertes Phenyl oder gegebenenfalls durch Alkyl mit 1 oder 2 Kohlenstoffatomen und/oder Halogen substituiertes Phenoxy,
X für Wasserstoff, Chlor oder Brom steht und
$R^1$ für Hydroxy, Alkoxy mit 1 bis 6 Kohlenstoffatomen, Alkyl mit 1 bis 6 Kohlenstoffatomen, Fluor, Chlor Brom oder Iod steht.

2. Fluorcyclopropyl-Derivate der Formel (I) gemäß Anspruch 1, in denen
R für Methyl, Ethyl, Isopropyl, tert.-Butyl
oder für Benzyl staht, das im Phenylteil einfach oder zweifach, gleichartig oder verschieden substituiert sein kann durch Fluor, Chlor und/oder Methyl,
X für Wasserstoff, Chlor oder Brom steht und
$R^1$ für Hydroxy, Alkoxy mit 1 bis 4 Kohlenstoffatomen, Alkyl mit 1 bis 4 Kolhlenstoffatomen; Fluor, Chlor, Brom oder Iod steht.

3. Fluorcyclopropyl-Derivat gemäß Anspruch 1, gekennzeichnet durch die Formel

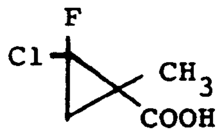

(I-1)

4. Fluorcyclopropyl-Derivat gemäß Anspruch 1, gekennzeichnet durch die Formel

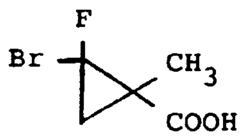

(I-2)

5. Fluorcyclopropyl-Derivat gemäß Anspruch 1, gekennzeichnet durch die Formel

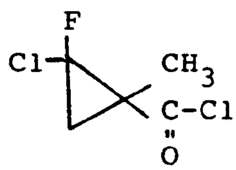

(I-3)

6. Fluorcyclopropyl-Derivat gemäß Anspruch 1, gekennzeichnet durch die Formel

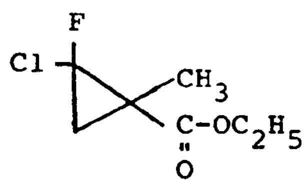

(I-4)

7. Fluorcyclopropyl-Derivat gemäß Anspruch 1, gekennzeichnet durch die Formel

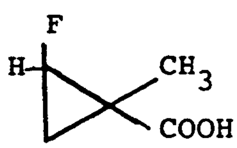

(I-5).

8. Verfahren zur Herstellung von Fluocyclopropyl-Derivaten der Formel

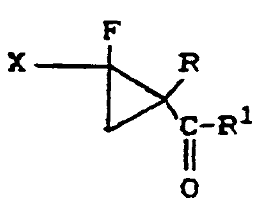

(I)

in welcher

R für Alkyl mit 1 bis 4 Kohlenstoffatomen oder für Benzyl steht, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1

oder 2 Kohlenstoffatomen und 1 bis 5 gleichen oder verschiedenen Halogenatomen, Halogenalkoxy mit 1 oder 2 Kohlenstoffatomen

und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis

4 Kohlenstoffatomen im Alkylteil, gegebenenfalls durch Alkyl mit 1 oder 2 Kohlenstoffatomen und/ oder Halogen substituiertes Phenyl oder gegebenenfalls durch Alkyl mit 1 oder 2 Kohlenstoffatomen und/ oder Halogen substituiertes Phenoxy,

X für Wasserstoff, Chlor oder Brom steht und

$R^1$ für Hydroxy, Alkoxy mit 1 bis 6 Kohlenstoffatomen, Alkyl mit 1 bis 6 Kohlenstoffatomen, Fluor, Chlor Brom oder Iod steht,

dadurch gekennzeichnet, daß man

a) Vinyl-cyclopropan-Derivate der Formel

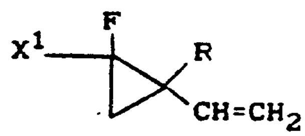

(II)

in welcher

R die oben angegebene Bedeutung hat und

$X^1$ für Chlor oder Brom steht,

mit starken Oxidationsmitteln in Gegenwart eines Verdünnungsmittels umsetzt,

oder

b) Fluorcyclopropyl-Derivate der Formel

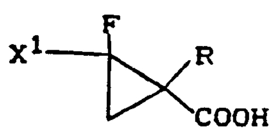

(Ia)

in welcher

R und $X^1$ die oben angegebene Bedeutung haben,

entweder

α) mit einem Halogenierungsmittel gegebenenfalls in Gegenwart eines Verdünnungsmittels umsetzt,

oder

β) mit Alkoholen der Formel

$R^2$-OH (III)

in welcher

$R^2$ für Alkyl mit 1 bis 6 kohlenstoffatomen steht,

gegebenenfalls in Gegenwart eines Katalysators und gegebenenfalls in Gegenwart eines Verdünnungsmittels umsetzt,

oder

γ) mit Wasserstoff in Gegenwart eines Katalysators und in Gegenwart eines Amins sowie in Gegenwart eines Verdünnungsmittels umsetzt,

oder

c) Fluorcyclopropyl-Derivate der Formel

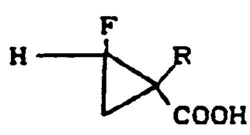

(Ib)

in welcher

R die oben angegebene Bedeutung hat,

mit einem Halogenierungsmittel gegebenenfalls in Gegenwart eines Vordünnungsmittels umsetzt,

oder

d) Fluorcyclopropyl-Derivate der Formel

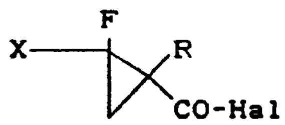

(Ic)

in welcher

R und X die oben angegebene Bedeutung haben und

Hal für Fluor, Chlor oder Brom steht,

mit Alkoholen der Formel

$R^2$-OH (III)

in welcher

$R^2$ die oben angegebene Bedeutung hat,

gegebenenfalls in Gegenwart eines Katalysators und gegebenenfalls in Gegenwart eines Verdünnungsmittels umsetzt,

oder

e) Fluorcyclopropyl-Derivate der Formel

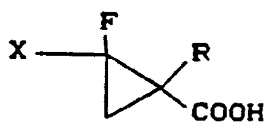

_ (Id)

in welcher

R und X die oben angegebene Bedeutung haben,

mit metallorganischen Verbindungen der Formel

$R^3$-Li (IV)

in welcher

$R^3$ für Alkyl mit 1 bis 6 kohlenstoffatomen steht,

in Gegenwart eines Verdünnungsmittels umsetzt.

## Claims

1. Fluorocyclopropyl derivatives of the formula

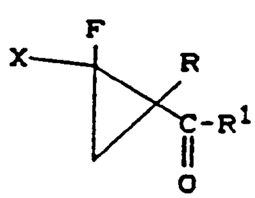

(I)

in which

R represents alkyl having 1 to 4 carbon atoms or represents benzyl which can be monosubstituted to trisubstituted in the phenyl moiety by identical or different substituents from the series comprising halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio

having 1 or 2 carbon atoms and 1 to 5 identical or different halogen atoms, alkoximinoalkyl having 1 to 4 carbon atoms in the alkyl moiety and 1 to 4 carbon atoms in the alkoxy moiety, phenoximinoalkyl which has 1 to 4 carbon atoms in the alkyl moiety and which is optionally substituted by alkyl having 1 or 2 carbon atoms and/or halogen, phenyl which is optionally substituted by alkyl having 1 or 2 carbon atoms and/or halogen, or by phenoxy which is optionally substituted by alkyl having 1 or 2 carbon atoms and/or halogen,

X represents hydrogen, chlorine or bromine and

$R^1$ represents hydroxyl, alkoxy having 1 to 6 carbon atoms, alkyl having 1 to 6 carbon atoms, fluorine, chlorine, bromine or iodine.

2. Fluorocyclopropyl derivatives of the formula (I) according to Claim 1 in which

R represents methyl, ethyl, isopropyl, tert.-butyl or for benzyl which can be monosubstituted or disubstituted in the phenyl moiety by identical or different substituents from the series comprising fluorine, chlorine and/or methyl,

X represents hydrogen, chlorine or bromine and

$R^1$ represents hydroxyl, alkoxy having 1 to 4 carbon atoms, alkyl having 1 to 4 carbon atoms, fluorine, chlorine, bromine or iodine.

3. Fluorocyclopropyl derivative according to Claim 1, characterised by the formula

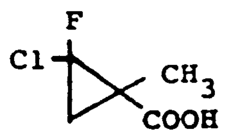

(I-1)

4. Fluorocyclopropyl derivative according to Claim 1, characterised by the formula

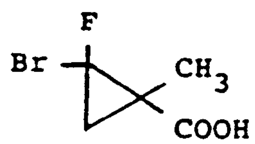

(I-2)

5. Fluorocyclopropyl derivative according to Claim 1, characterised by the formula

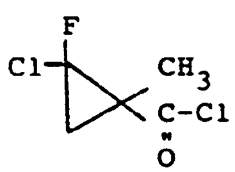

(I-3)

6. Fluorocyclopropyl derivative according to Claim 1, characterised by the formula

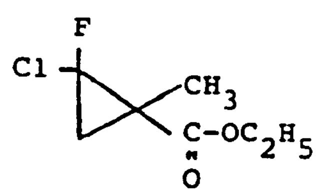

(I-4)

7. Fluorocyclopropyl derivative according to Claim 1, characterised by the formula

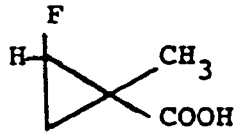

(I-5)

8. Process for the preparation of fluorocyclopropyl derivatives of the formula

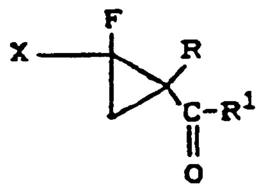

(I)

R represents alkyl having 1 to 4 carbon atoms or represents benzyl which can be monosubstituted to trisubstituted in the phenyl moiety by identical or different substituents from the series comprising halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, alkoximinoalkyl having 1 to 4 carbon atoms in the alkyl moiety and 1 to 4 carbon atoms in the alkoxy moiety, phenoximinoalkyl which has 1 to 4 carbon atoms in the alkyl moiety and which is optionally substituted by alkyl having 1 or 2 carbon atoms and/or halogen, phenyl which is optionally substituted by alkyl having 1 or 2 carbon atoms and/or halogen, or by phenoxy which is optionally substituted by alkyl having 1 or 2 carbon atoms and/or halogen, characterised in that

a) vinyl-cyclopropane derivatives of the formula

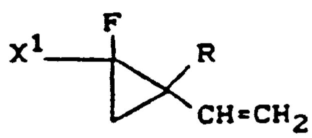

(II)

in which

R has the abovementioned meaning and

$X^1$ represents chlorine or bromine,

are reacted with strong oxidants in the presence of a diluent, or

b) fluorocyclopropyl derivatives of the formula

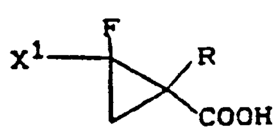

(Ia)

in which

R and $X^1$ have the abovementioned meaning are reacted either

α) with a halogenating agent, if appropriate in the presence of a diluent,

or

β) with alcohols of the formula

$R^2$-OH (III)

in which

$R^2$ represents alkyl, having 1 to 6 carbon atoms,

if appropriate in the presence of a catalyst and

if appropriate in the presence of a diluent,

or

γ) with hydrogen in the presence of a catalyst and in the presence of an amine and in the presence of a diluent,

or

c) fluorocyclopropyl derivatives of the formula

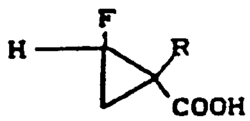

(Ib)

in which

R has the abovementioned meaning is reacted with a halogenating agent, if appropriate in the presence of a diluent,

or

d) fluorocyclopropyl derivatives of the formula

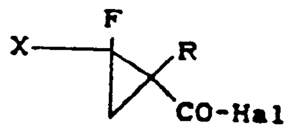

(Ic)

in which

R and X have the abovementioned meaning and

Hal represents fluorine, chlorine or bromine,

are reacted with alcohols of the formula

$R^2$-OH (III)

in which

$R^2$ has the abovementioned meaning,

if appropriate in the presence of a catalyst and

if appropriate in the presence of a diluent,

or

e) fluorocyclopropyl derivatives of the formula

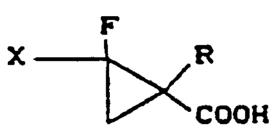

(Id)

in which

R and X have the abovementioned meaning are reacted with organometal compounds of the formula

$R^3$-Li (IV)

in which

$R^3$ represents alkyl having 1 to 6 carbon atoms

in the presence of a diluent.

## Revendications

1. Dérivés de fluorocyclopropyle de formule

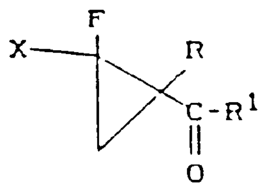

(I)

dans laquelle

R représente un groupe alkyle contenant 1 à 4 atomes de carbone ou un groupe benzyle qui peut être substitué dans la fraction phényle 1 à 3 fois, de manière identique ou différente, par un atome d'halogène, par un groupe alkyle contenant 1 à 4 atomes de carbone, par un groupe alcoxy contenant 1 à 4 atomes de carbone, par un groupe alkylthio contenant 1 à 4 atomes de carbone, par un groupe halogénalkyle contenant 1 ou 2 atomes de carbone et 1 à 5 atomes d'halogène identiques ou différents, par un groupe halogénalcoxy contenant 1 ou 2 atomes de carbone et 1 à 5 atomes d'halogène identiques ou différents, par un groupe halogénalkylthio contenant 1 ou 2 atomes de carbone et 1 à 5 atomes d'halogène identiques ou différents, par un groupe alcoximinoalkyle contenant 1 à 4 atomes de carbone dans la fraction alkyle et 1 à 4 atomes de carbone dans la fraction alcoxy, un groupe phénoximinoalkyle contenant 1 à 4 atomes de carbone dans la fraction alkyle, éventuellement substitué par un groupe alkyle contenant 1 ou 2 atomes de carbone et/ou par un halogène, un groupe phényle éventuellement substitué par un groupe alkyle contenant 1 ou 2 atomes de carbone et/ou par un halogène ou encore un groupe phénoxy éventuellement substitué par un groupe alkyle contenant 1 ou 2 atomes de carbone et/ou par un atome d'halogène,

X représente l'hydrogène, le chlore ou le brome, et

$R^1$ représente un groupe hydroxyle, un groupe alcoxy contenant 1 à 6 atomes de carbone, un groupe alkyle contenant 1 à 6 atomes de carbone, le fluor, le chlore, le brome ou l'iode.

2. Dérivés de fluorocyclopropyle de formule (I) selon la revendication 1, dans lesquels

R représente un groupe méthyle, éthyle, isopropyle ou tert.-butyle,

ou un groupe benzyle, qui peut être substitué dans la fraction phényle 1 à 2 fois, de manière identique ou différente, par le fluor, le chlore et/ou par un groupe méthyle,

X représente l'hydrogène, le chlore ou le brome, et

$R^1$ représente un groupe hydroxyle, un groupe alcoxy contenant 1 à 4 atomes de carbone, un groupe alkyle contenant 1 à 4 atomes de carbone, le fluor, le chlore, le brome ou l'iode.

3. Dérivé de fluorocyclopropyle selon la revendication 1, caractérisé par la formule

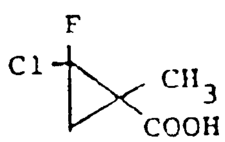

(I-1)

4. Dérivé de fluorocyclopropyle selon la revendication 1, caractérisé par la formule

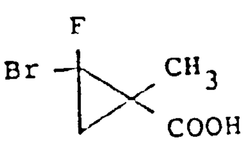

(I-2)

5. Dérivé de fluorocyclopropyle selon la revendication 1, caractérisé par la formule

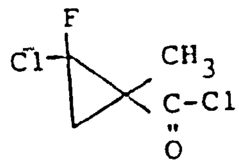

(I-3)

6. Dérivé de fluorocyclopropyle selon la revendication 1, caractérisé par la formule

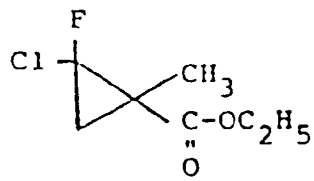

(I-4)

7. Dérivé de fluorocyclopropyle selon la revendication 1, caractérisé par la formule

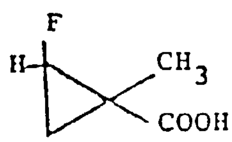

(I-5) .

8. Procédé de préparation de dérivés de fluorocyclopropyle de formule

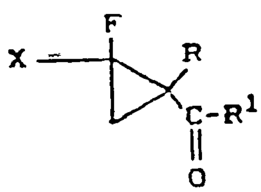

(I)

dans laquelle

R représente un groupe alkyle contenant 1 à 4 atomes de carbone ou un groupe benzyle qui peut être substitué dans la fraction phényle 1 à 3 fois, de manière identique ou différente, par un halogène, par un groupe alkyle contenant 1 à 4 atomes de carbone, par un groupe alcoxy contenant 1 à 4 atomes de carbone, par un groupe alkylthio contenant 1 à 4 atomes de carbone, par un groupe halogénalkyle contenant 1 ou 2 atomes de carbone et 1 à 5 atomes d'halogène identiques ou différents, par un groupe halogénalcoxy contenant 1 ou 2 atomes de carbone et 1 à 5 atomes d'halogène identiques ou différents, par un groupe halogénalkylthio contenant 1 ou 2 atomes de carbone et 1 à 5 atomes d'halogène identiques ou différents, par un groupe alcoximinoalkyle contenant 1 à 4 atomes de carbone dans la fraction alkyle et 1 à 4 atomes de carbone dans la fraction alcoxy, un groupe phénoximinoalkyle contenant 1 à 4 atomes de carbone dans la fraction alkyle, éventuellement substitué par un groupe alkyle contenant 1 ou 2 atomes de carbone et/ou par un halogène, un groupe phényle éventuellement substitué par un groupe alkyle contenant 1 ou 2 atomes de carbone et/ou par un halogène ou encore un groupe phénoxy éventuellement substitué par un groupe alkyle contenant 1 ou 2 atomes de carbone et/ou par un halogène,

X représente l'hydrogène, le chlore ou le brome, et

$R^1$ représente un groupe hydroxyle, un groupe alcoxy contenant 1 à 6 atomes de carbone, un groupe alkyle contenant 1 à 6 atomes de carbone, le fluor, le chlore, le brome ou l'iode,

caractérisé en ce que

a) des dérivés de vinylcyclopropane de formule

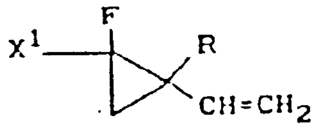

(II)

dans laquelle
R a la signification mentionnée ci-dessus et
$X^1$ représente le chlore ou le brome,
sont amenés à réagir avec des oxydants forts en présence d'un diluant
ou bien
b) des dérivés de fluorocyclopropyle de formule

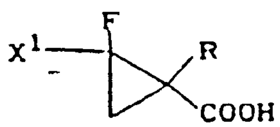

(Ia)

dans laquelle
R et $X^1$ ont la signification mentionnée ci-dessus, sont amenés à réagir
soit
α) avec un agent d'halogénation éventuellement en présence d'un diluant,
soit
β) avec des alcools de formule

$R^2$-OH (III)

dans laquelle
$R^2$ représente un groupe alkyle contenant 1 à 6 atomes de carbone,
éventuellement en présence d'un catalyseur et éventuellement en présence d'un diluant,
ou encore
γ) avec de l'hydrogène en présence d'un catalyseur et en présence d'une amine, ainsi qu'en présence d'un diluant,
ou bien
c) des dérivés de fluorocyclopropyle de formule

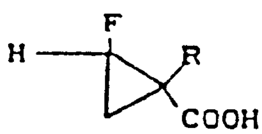

(Ib)

R a la signification mentionnée ci-dessus,
sont amenés à réagir avec un agent d'halogénation et éventuellement en présence d'un diluant
ou bien
d) des dérivés de fluorocyclopropyle de formule

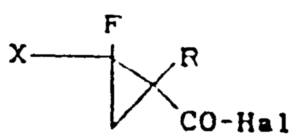

(Ic)

dans laquelle
R et X ont la signification mentionnée ci-dessus et
Hal représente le fluor, le chlore ou le brome
sont amenés à réagir avec des alcools de formule

$R^2$-OH (III)

dans laquelle
$R^2$ a la signification mentionnée ci-dessus
éventuellement en présence d'un catalyseur et éventuellement en présence d'un diluant,
ou bien
e) des dérivés de fluorocyclopropyle de formule

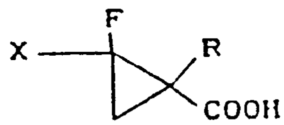

(Id)

dans laquelle
R et X ont la signification mentionnée ci-dessus,
sont amenés à réagir avec des composés organométalliques de formule

$R^3$-Li (IV)

dans laquelle
$R^3$ représente un groupe alkyle contenant 1 à 6 atomes de carbone,
en présence d'un diluant.